# EUROPEAN PATENT APPLICATION

(11) **EP 2 845 541 A1**
(43) Date of publication of application: **11.03.2015**
(21) Application number: 14165676.9
(22) Date of filing: 23.04.2014
(51) Int. Cl.: A61B 8/00

(54) **Probe for ultrasonic diagnostic apparatus**

(30) Priority: 29.08.2013 KR 20130103005
(71) Applicant: Samsung Medison Co., Ltd., Gangwon-do 250-870 (KR)
(72) Inventor: Lee, Won Hee, 105-1506 Seoul (KR); Lee, Ju Young, Incheon (KR); Song, Kyung Hun, 102-503 Gyeonggi-do (KR); Lee, Sung Jae, 405-30 Seoul (KR); Jang, Won Seok, 306-2301 Seoul (KR); Hwang, Won Soon, Hanam-si 403-203 Gyeonggi-do (KR)
(74) Representative: Schmid, Wolfgang

(57) **Abstract**

Disclosed is a probe for an ultrasonic diagnostic apparatus with an improved heat-radiation structure using graphene. The probe includes a case to form an exterior appearance, a piezoelectric layer provided in the case to generate ultrasonic waves, a backing layer provided at the rear of the piezoelectric layer to prevent the ultrasonic waves from being transmitted backward from the piezoelectric layer, and a heat-radiation unit to radiate heat transmitted from the piezoelectric layer to the outside the case. The heat-radiation unit includes graphene. By attaching graphene having a high thermal conductivity to a heat source of the probe, a surface temperature of the heat source is reduced.

## Description

### CROSS-REFERENCE TO RELATED APPLICATION

This application claims the benefit of Korean Patent Application No. 2013-0103005, filed on August 29, 2013 in the Korean Intellectual Property Office, the disclosure of which is incorporated herein by reference.

### BACKGROUND

### 1. Field

Embodiments of the present invention relate to a probe for an ultrasonic diagnostic apparatus with an improved heat-radiation structure using graphene.

### 2. Description of the Related Art

In general, ultrasonic diagnostic apparatuses direct ultrasonic signals from a body surface of an object to a desired region inside a body, and obtain an image related to a monolayer of soft tissue or the bloodstream using the ultrasonic signals reflected from the desired region. The ultrasonic diagnostic apparatuses are relatively small and cheap compared to other diagnostic apparatuses such as X-ray machines, computerized tomography scanners, magnetic resonance imaging scanners, nuclear medicine scanners and the like. The ultrasonic diagnostic apparatuses also have features of displaying images in real time and being highly safe without radiation exposure that may occur in X-ray machines or the like. The ultrasonic diagnostic apparatuses are widely used to examine internal organs such as the heart, abdominal areas, reproductive organs, and gynecological problems.

An ultrasonic diagnostic apparatus includes probes to transmit ultrasonic signals to an object to be examined and receive echo signals reflootod from the object, to thereby obtain an ultrasonic image of the object. Recently, research and development to make highly efficient, much smaller and much lighter probes are being actively carried out.

The current trend is to manufacture smaller probes, however, heat-radiation is a major obstacle. Because the probe has a sealed structure, it is hard to realize a fan-type heat radiation. In addition, a sufficient heat-radiation effect is not obtained by a heat-radiation material made from general metals or alloys.

Since a piezoelectric element used for a probe has poor heat tolerance, a functional error may occur when continuously exposed to high temperatures, which may cause malfunction and durability deterioration of the probe. Further, since the probe is used in close contact with an object to be examined, especially human skin, the probe should operate within a certain temperature limit. Therefore, in order to miniaturize probes, the heat radiation problems must be resolved.

### SUMMARY

It is an aspect of the present invention to provide a probe for an ultrasonic diagnostic apparatus with an improved heat-radiation structure using graphene.

It is another aspect of the present invention to provide a probe for an ultrasonic diagnostic apparatus capable of blocking electromagnetic waves as well as enhancing heat-radiation effect by attaching graphene to a heat source and a printed circuit board.

Additional aspects of the invention will be set forth in part in the description which follows and, in part, will be obvious from the description, or may be learned by practice of the invention.

In accordance with one aspect of the present invention, a probe for an ultrasonic diagnostic apparatus includes a case to form an exterior appearance, a piezoelectric layer provided in the case to generate ultrasonic waves, a backing layer provided at the rear of the piezoelectric layer to prevent the ultrasonic waves from being transmitted backward from the piezoelectric layer, and a heat-radiation unit to radiate heat transmitted from the piezoelectric layer to the outside of the case. The heat-radiation unit includes graphene.

The heat-radiation unit may be disposed adjacent to the backing layer.

The heat-radiation unit may be attached to an outer surface of the backing layer.

The heat-radiation unit may be formed in a plate shape.

The heat-radiation unit may extend to cover a printed circuit board disposed beneath the backing layer so as to block electromagnetic waves.

The heat-radiation unit may be inserted into the backing layer.

The heat-radiation unit may include plural plates which are spaced apart from each other.

The heat-radiation unit may include plural plates which are arranged perpendicular to each other in a grid pattern.

The heat-radiation unit may include plural plates which are radially arranged.

As described above, by attaching graphene having a high thermal conductivity to a heat source of the probe, a surface temperature of the heat source is reduced.

Further, in addition to the effective heat radiation, electromagnetic waves may be blocked by attaching graphene to a printed circuit board as well as a heat source by virtue of the electromagnetic-shielding features of graphene.

### BRIEF DESCRIPTION OF THE DRAWINGS

These and/or other aspects of the invention will become apparent and more readily appreciated from the following description of the embodiments, taken in conjunction with the accompanying drawings of which:
FIG. 1 is a view showing an ultrasonic diagnostic apparatus according to an embodiment of the present invention;
FIG. 2 is a view showing a probe for an ultrasonic diagnostic apparatus according to the embodiment of the present invention;
FIG. 3 is a view showing a probe for an ultrasonic diagnostic apparatus according to a first embodiment of the present invention;
FIG. 4 is a view showing a probe for an ultrasonic diagnostic apparatus according to a second embodiment of the present invention;
FIG. 5 is a view showing a probe for an ultrasonic diagnostic apparatus according to a third embodiment of the present invention; and
FIG. 6 is a view showing a probe for an ultrasonic diagnostic apparatus according to a fourth embodiment of the present invention.

### DETAILED DESCRIPTION

Reference will now be made in detail to the embodiments of the present invention, examples of which are illustrated in the accompanying drawings, wherein like reference numerals refer to like elements throughout.

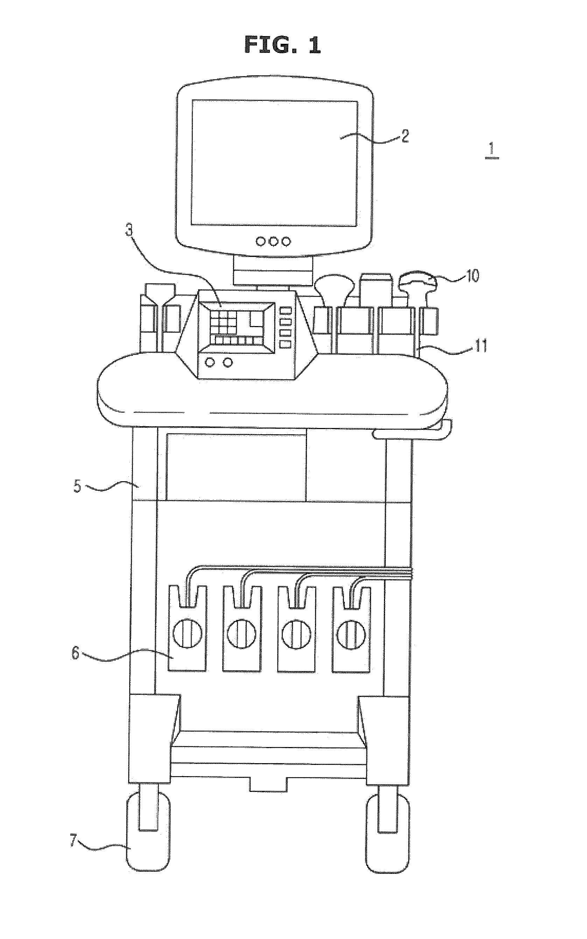

FIG. 1 is a view showing an ultrasonic diagnostic apparatus according to an embodiment of the present invention.

As shown in FIG. 1, an ultrasonic diagnostic apparatus 1 according to an embodiment of the present invention includes a housing 5 configured to generate an image of an object to be examined. A control panel 3 and a display unit 2 to display an image generated based upon echo signals reflected from the object may be mounted to the housing 5.

The ultrasonic diagnostic apparatus may further include a variety of probes 10 to transmit an ultrasonic signal to an object to be examined and receive an echo signal reflected from the object. The probes 10 may be electrically connected to the housing 5 through cables 11 integrally provided at the probes 10 and connectors 6.

Support units 7 are mounted to a bottom of the housing 5 to support the ultrasonic diagnostic apparatus 1. Each of the support units 7 may include a moving element, such as a wheel, to enable a user to move the ultrasonic diagnostic apparatus 1.

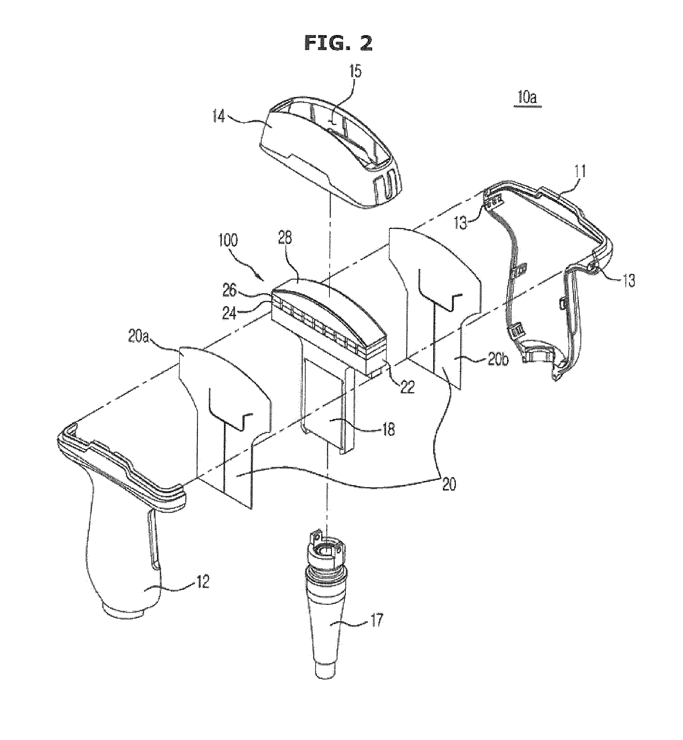

FIG. 2 is a view showing constitutional elements of a probe 10a for an ultrasonic diagnostic apparatus according to an embodiment of the present invention.

A probe 10a includes a main body 100 to transform signals, cases 11 and 12 and a cover 14 to surround the main body 100, and a handle 17 to be grabbed by an operator.

The cases 11 and 12 may include a first case 11 and a second case 12 which are configured to be coupled to each other to cover the lateral surfaces of the main body 100. The first and second cases 11 and 12 have shapes corresponding to each other. Hereinafter, the structure of the first case 11 will be described. The first case 11 is formed in a convex shape to have an accommodation space thereinside.

The first case 11 has an upper portion which is formed widely enough to accommodate the main body 100, and an upper surface which is formed to be coupled with the cover 14. The first case 11 is further provided with plural hooks 13 along the lateral surface which is in contact with the second case 12, thereby engaging the first case 11 with the second case 12. The first case 11 also has a lower portion which is shaped to form an opening when engaged with the second case 12, into which the handle 17 is inserted.

The cover 14 is engaged with the upper surfaces of the first and second cases 11 and 12, and covers the upper portion of the main body 100. The cover 14 may be formed with an opening 15 through which the top surface of the main body 100 is exposed outside. The top surface of the main body 100 exposed through the opening 15 comes into contact with a surface of an object to be diagnosed.

The main body 100 may includes a piezoelectric layer 24 to generate ultrasonic waves, a backing layer 22 to prevent the ultrasonic waves from being transmitted backward from the piezoelectric layer 24, a matching layer 26 disposed on the piezoelectric layer 24, and an acoustic lens 28 disposed on the matching layer 26. A printed circuit board (PCB) 18, which is electrically connected to electrode units provided at both lateral surfaces of the piezoelectric layer 24, may be disposed beneath the backing layer 22.

The electrode units may be made of a highly conductive metal such as gold, silver and copper, or graphite. The PCB may be configured as a flexible printed circuit board (FPCB) capable of supplying signals and electricity.

The piezoelectric layer 24 is made of a piezoelectric material capable of receiving electric signals, converting the signals into physical vibration and generating ultrasonic waves. A piezoelectric material is generally defined as a material having piezoelectric effect and converse piezoelectric effect, where it generates voltage if subjected to physical stress and generates physical deformation if voltage is applied thereto. In other words, a piezoelectric material means a material capable of converting electric energy into physical vibration and converting physical vibration into electric energy.

The piezoelectric material of the piezoelectric layer 24 may includes PZMT single crystal made from a solid solution of Lead Zirconate Titanate (PZT) ceramic, Magnesium Niobate and Titanate. Alternatively, the piezoelectric material of the piezoelectric layer 24 may include PZNT single crystal made from a solid solution of Zinc Niobate and Titanate.

The matching layer 26 is disposed on the piezoelectric layer 24. The matching layer 26 serves to reduce a difference in acoustic impedance between the piezoelectric layer 24 and an object to be examined so that the ultrasonic waves generated from the piezoelectric layer 24 are effectively transmitted to the object. The matching layer 26 may be configured as one or more layers. The matching layer 26 and the piezoelectric layer 24 may be split into a plurality of units, each of which has a certain width, through a dicing process.

Although not illustrated in the drawings, a protective layer may be disposed on the matching layer 26. The protective layer serves to prevent outward flow of high-frequency components, which may be generated from the piezoelectric layer 24, and to block inflow of external high-frequency signals. In order to protect internal components from water and chemicals used for sterilization, the protective layer may be made by coating or depositing a conductive material on a surface of a waterproof and chemically resistant film.

The acoustic lens 28, which is disposed on the matching layer 26, comes into direct contact with an object to be examined. The acoustic lens 28 may be shaped convex in the direction of the radiation of ultrasonic waves in order to focus the ultrasonic waves. If the speed of sound of the material of the acoustic lens 28 is lower than the speed of sound in the human body, the acoustic lens 28 may be concave. In this embodiment, as shown in FIG. 2, the acoustic lens 28 is convex in the direction of the radiation of ultrasonic waves.

The backing layer 22 is disposed beneath the piezoelectric layer 24. The backing layer 22 serves to absorb ultrasonic waves generated from the piezoelectric layer 24 and block the downward flow of the ultrasonic waves from the piezoelectric layer 24, thereby preventing image distortion. The backing layer 22 may be configured as plural layers in order to improve the effect of attenuating or blocking the ultrasonic waves.

The backing layer 22 is made from an acoustic backing material capable of absorbing the ultrasonic waves generated from the piezoelectric layer 24. The acoustic backing material may be made by combining metal powders (e.g., tungsten, copper and aluminum), ceramics and carbon allotrope powders using an epoxy resin, and may include rubber. Especially, metals having a high attenuation coefficient may be used for the acoustic backing material.

The processes of generating and receiving the ultrasonic waves of the probe 10a inevitably cause vibration of the piezoelectric layer 24 and heat associated therewith. Further, as probes have become smaller and smaller, the probes become highly integrated, and accordingly the amount of heat generation has increased.

Such heat may not be radiated outside, but transmitted to the acoustic lens 28 of the probe 10a. Because the acoustic lens 28 is an element directly contacting the patient's skin, the internal heat of the probe 10a may be transmitted to the patient's skin and cause a burn. In addition, the heat may cause functional disorder of the components of the probe 10a, which may result in negative influence on patient safety and diagnostic images. Accordingly, the probe 10a is required to have a structure capable of effectively radiating the heat outside.

From such a point of view, the probe 10a may include a heat-radiation unit 20 to radiate the internal heat outside. The heat-radiation unit 20 may include graphene having a high thermal conductivity.

Graphene is the thinnest layer stripped off from graphite that consists of carbon atoms piled up in a hexagonal beehive shape. Similarly to carbon nanotube (CNT), graphene is a nanomaterial consisting of a single layer of carbon atoms whose atomic number is 6. Graphene has a two-dimensional plane shape with a thickness of 0.2 nm, and has high physical and chemical stabilities. It is also known that graphene conducts electricity over 100 times better than copper and electrons travel over 100 times faster in graphene than in single crystal silicon primarily used for semiconductors. Further, the thermal conductivity of graphene is about 5000 W/mK, which is over twice that of diamond.

The heat-radiation unit 20 may be positioned adjacent to the backing layer 22 disposed beneath the piezoelectric layer 24 which may be called a heat source of the probe 10a. The heat-radiation unit 20 positioned adjacent to the backing layer 22 may be arranged so as to radiate the heat transmitted to the backing layer 22 from the piezoelectric layer 24 to the outside of the cases 11 and 12.

The heat-radiation unit 20 may be attached to a lateral surface of the main body 100 including the backing layer 22. The heat-radiation unit 20 may be a thin plate which has the same shape as the lateral surface of the main body 100. As shown in FIG. 2, the heat-radiation unit 20 may include a first heat-radiation element 20a and a second heat-radiation element 20b, which are respectively attached to both lateral surfaces of the main body 100.

Because the thin plate-shaped first and second heat-radiation elements 20a and 20b are in close contact with the main body 100, an additional space for the first and second heat-radiation elements 20a and 20b inside the cases 11 and 12 may be unnecessary. Further, since the heat-radiation unit 20 has a size corresponding to the whole area of the lateral surface of the main body 100, a heat radiation area may be enlarged and accordingly heat radiation efficiency may be increased.

So as to block electromagnetic waves, the heat-radiation unit 20 may have a size sufficient to cover the PCB 18 disposed beneath the backing layer 22. An additional device to block electromagnetic waves from the PCB has been necessary in conventional probes for ultrasonic diagnostic apparatuses. However, both heat-radiation and electromagnetic-shielding problems are simultaneously solved in the probe of the present invention by attaching graphene having electromagnetic shielding properties to the whole area of the main body 100.

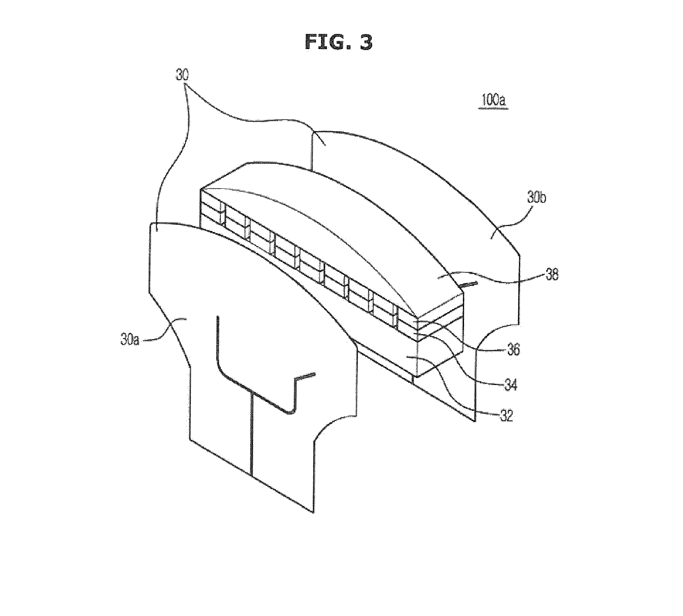

In order to clearly describe a variety of embodiments of the heat-radiation unit disposed adjacent to the backing layer, the illustration of the other components than the backing layer, the piezoelectric layer, the matching layer and the acoustic lens are omitted in FIGS. 3 through 6.

FIG. 3 is a view showing a probe for an ultrasonic diagnostic apparatus according to a first embodiment of the present invention.

As shown in the drawing, a heat-radiation unit 30 may be attached to outer surfaces of a main body 100a of a probe comprising an acoustic lens 38, a matching layer 36, a piezoelectric layer 34 and a backing layer 32. The heat-radiation unit 30 may include a first heat-radiation element 30a and a second heat-radiation element 30b which are respectively attached to both lateral surfaces of the main body 100a.

The first heat-radiation element 30a and the second heat-radiation element 30b may be formed in a plate shape capable of being closely attached to the surface of the main body 100a. Differently from the heat-radiation unit 20 depicted in FIG. 2, the heat-radiation unit 30 in FIG. 3 may be formed not to extend to a lower portion of the main body 100a. That is, graphene is attached only to the region requiring heat radiation. Accordingly, waste of materials is reduced.

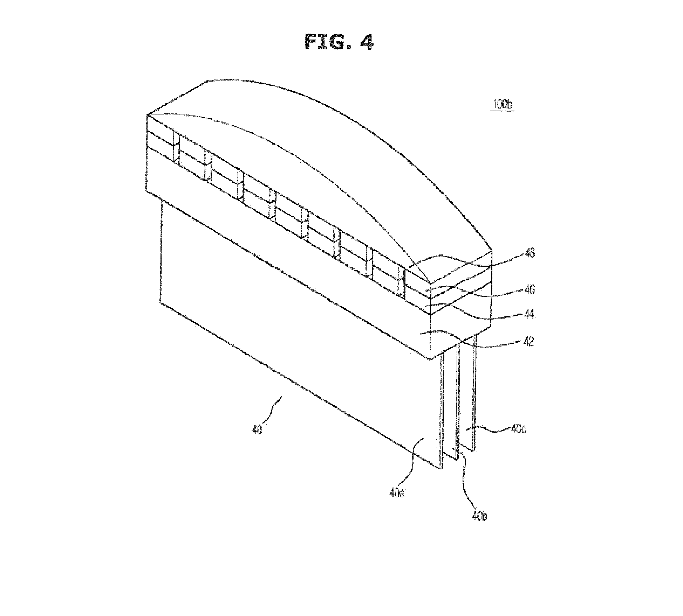

FIG. 4 is a view showing a probe for an ultrasonic diagnostic apparatus according to a second embodiment of the present invention.

As shown in the drawing, a heat-radiation unit 40 may be inserted into a main body 100b of a probe comprising an acoustic lens 48, a matching layer 46, a piezoelectric layer 44 and a backing layer 42. The inserted portion of the heat-radiation unit 40 may be fixed by silicon filled in the backing layer 42. The heat-radiation unit 40 may include plural plates which are spaced apart from each other. The plural plates are made from graphene so as to radiate heat to the outside.

The heat-radiation unit 40 depicted in FIG. 4 includes a first heat-radiation element 40a, a second heat-radiation element 40b and a third heat-radiation element 40c which are spaced apart from each other. Although three plates are illustrated in the drawing as the heat-radiation unit 40, the number of the plates is not limited to three. The heat-radiation unit 40 may include a proper number of plates to secure spaces for more efficient heat radiation.

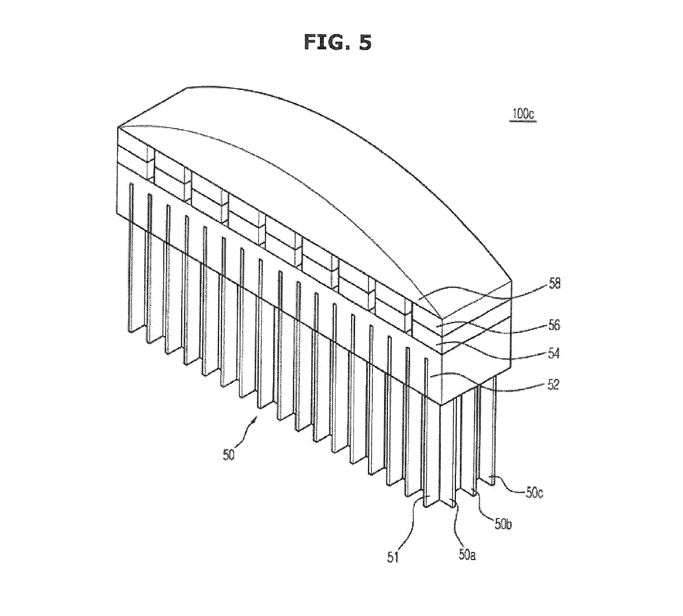

FIG. 5 is a view showing a probe for an ultrasonic diagnostic apparatus according to a third embodiment of the present invention.

As shown in the drawing, a heat-radiation unit 50 may be inserted into a main body 100c of a probe comprising an acoustic lens 58, a matching layer 56, a piezoelectric layer 54 and a backing layer 52. In addition to plural plates 50a, 50b and 50c, which are identical to the plates 40a, 40b and 40c of the heat-radiation unit 40 depicted in FIG. 4, the heat-radiation unit 50 in this embodiment may further include plural plates 51 which are spaced apart from each other and arranged perpendicular to the plates 50a, 50b and 50c.

The perpendicularly-arranged plates 50a, 50b, 50c and 51 are made from graphene so as to radiate heat to the outside. When viewed from above, the perpendicularly-arranged plates 50a, 50b, 50c and 51 of the heat-radiation unit 50 are arranged in a grid pattern. The perpendicular arrangement of the plates 50a, 50b, 50c and 51 in a grid pattern may increase the heat-radiation area.

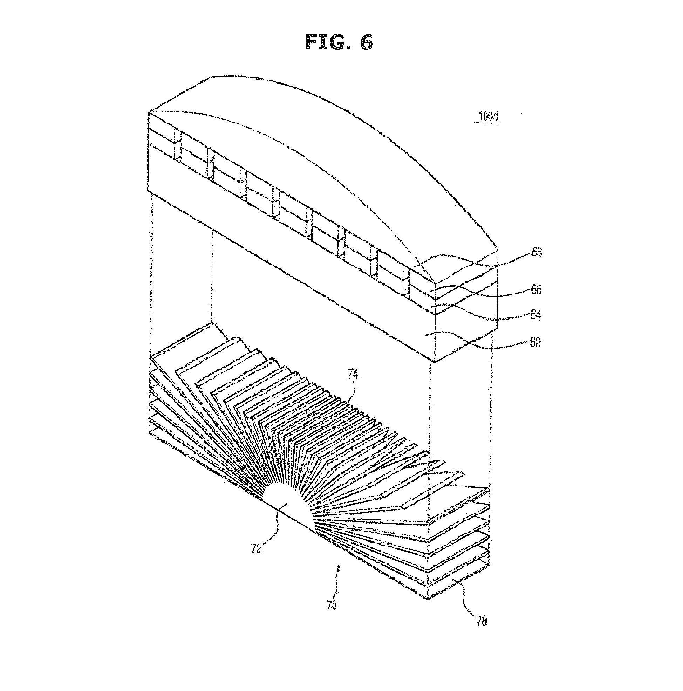

FIG. 6 is a view showing a probe for an ultrasonic diagnostic apparatus according to a fourth embodiment of the present invention.

As shown in the drawing, a heat-radiation unit 70 may be inserted into a main body 100d of a probe comprising an acoustic lens 68, a matching layer 66, a piezoelectric layer 64 and a backing layer 62. The heat-radiation unit 70 may include plural plates which are radially arranged. The plural plates of the heat-radiation unit 70 are made from graphene and extend in a radial direction from a center portion 72.

So as to fit into the main body 100d, an outer plate 78 horizontally extending from the center portion 72 may be arranged parallel to a floor, and a middle plate 74 upwardly extending from the center portion 72 may be the shortest of the plural plates. Each of the plural plates is arranged at a regular angle apart from the adjacent ones between the middle plate 74 and the outer plate 78.

The specific shapes of the probe and graphene attached to or inserted into the probe have been described with reference to the drawings, however these are illustrative only and other various shapes of graphene may be used to radiate heat from a probe for an ultrasonic diagnostic apparatus.

Although a few embodiments of the present invention have been shown and described, it would be appreciated by those skilled in the art that changes may be made in these embodiments without departing from the principles and spirit of the invention, the scope of which is defined in the claims and their equivalents.

## Claims

1. A probe for an ultrasonic diagnostic apparatus comprising:
a case to form an exterior appearance;
a piezoelectric layer provided in the case to generate ultrasonic waves;
a backing layer provided at the rear of the piezoelectric layer to prevent the ultrasonic waves from being transmitted backward from the piezoelectric layer; and
a heat-radiation unit to radiate heat transmitted from the piezoelectric layer to the outside the case,
wherein the heat-radiation unit includes graphene.

2. The probe for an ultrasonic diagnostic apparatus according to claim 1, wherein the heat-radiation unit is disposed adjacent to the backing layer.

3. The probe for an ultrasonic diagnostic apparatus according to claim 2, wherein the heat-radiation unit is attached to an outer surface of the backing layer.

4. The probe for an ultrasonic diagnostic apparatus according to claim 3, wherein the heat-radiation unit is formed in a plate shape.

5. The probe for an ultrasonic diagnostic apparatus according to claim 4, wherein the heat-radiation unit extends to cover a printed circuit board disposed beneath the backing layer so as to block electromagnetic waves.

6. The probe for an ultrasonic diagnostic apparatus according to claim 2, wherein the heat-radiation unit is inserted into the backing layer.

7. The probe for an ultrasonic diagnostic apparatus according to claim 6, wherein the heat-radiation unit includes plural plates which are spaced apart from each other.

8. The probe for an ultrasonic diagnostic apparatus according to claim 6, wherein the heat-radiation unit includes plural plates which are arranged perpendicular to each other in a grid pattern.

9. The probe for an ultrasonic diagnostic apparatus according to claim 6, wherein the heat-radiation unit includes plural plates which are radially arranged.
